Europäisches Patentamt

European Patent Office

Office européen des brevets

(11) Publication number: **0 066 759**
**B1**

# EUROPEAN PATENT SPECIFICATION

(45) Date of publication of patent specification: **11.09.85**

(51) Int. Cl.[4]: **A 01 N 35/02,** A 61 L 2/00

(21) Application number: **82104483.1**

(22) Date of filing: **21.05.82**

(54) Dialdehyde containing compositions.

(30) Priority: **21.05.81 US 266131**

(43) Date of publication of application:
**15.12.82 Bulletin 82/50**

(45) Publication of the grant of the patent:
**11.09.85 Bulletin 85/37**

(84) Designated Contracting States:
**AT BE CH DE FR GB IT LI LU NL SE**

(56) References cited:
**US-A-3 282 775**
**US-A-3 983 252**

**JOURNAL OF PHARMACEUTICAL SCIENCES,
vol. 53, no. 10, October 1964, pages 1273-1275,
Washington D.C., USA, P.M. BORICK et al.:
"Alkalinized glutaraldehyde, a new
antimicrobial agent"**

(73) Proprietor: **UNION CARBIDE CORPORATION
Old Ridgebury Road
Danbury Connecticut 06817 (US)**

(72) Inventor: **Eagar, Robert Gouldman, Jr.
3506 Carol Court
Yorktown Heights New York 10598 (US)**
Inventor: **Freid, Marvin George, Jr.
Brookdale Road
Putnam Valley New York 10579 (US)**

(74) Representative: **Schmied-Kowarzik, Volker, Dr.
et al
Patentanwälte Dr. V. Schmied-Kowarzik Dipl.-
Ing. G. Dannenberg Dr. P. Weinhold Dr. D. Gudel
Dipl.-Ing. S. Schubert Dr. P. Barz
Siegfriedstrasse 8
D-8000 München 40 (DE)**

Courier Press, Leamington Spa, England.

# 0 066 759

**Description**

This invention is directed to an aqueous composition comprising a saturated dialdehyde containing from 2 to 6 carbon atoms, an alkali metal bicarbonate and/or carbonate, and one or more additional buffers such that the initial aqueous solution has a pH of greater than 7 to about 9.0. These compositions are suitable for use as disinfectants and/or sporicides, particularly for laboratory, surgical, dental and other equipment.

Saturated dialdehydes, particularly glutaraldehyde, are widely used as sterilizers, biocides and disinfectants. Glutaraldehyde is more effective as a sterilant or microbiocide when used as an alkaline solution, since it exerts faster anti-microbal activity. It is commercially available as an aqueous acidic solution, which is sometimes alkalinated to a pH of from about 7.8 to about 8.5, since it most effective at such a pH range. The pH of the solution is generally maintained at from about 7.8 to about 8.5 by the use of buffers.

Glutaraldehyde solutions are stored in containers which may be opened and closed many times during use or the container may be left open while the glutaraldehyde solution is being used. It has been found that during such periods when the gluraldehyde solution is exposed to air, the pH changes or "drifts". If the pH of the solution changes so that it becomes acidic, its rate of microbiocidal activity becomes substantially slower. If the pH of the solution drifts to become more basic, the rate of polymerization of the glutaraldehyde accelerates, which results in a lowering of the effective amount of glutaraldehyde in solution, and hence a decrease in its biological activity.

Thus, there is a need to formulate the dialdehyde solution so as to minimize the pH drift.

U.S. Patent 3,016,328 describes a dialdehyde sporicidal composition comprising a mixture of a saturated dialdehyde, a lower alkanol, and an alkali metal carbonate or bicarbonate alkalinating agent. The examples in the patent describe the use of alkalinating agents such as alkali metal carbonates or bicarbonates. The patent also describes that the composition may be used without the lower alkanol which permits the addition of larger quantities of alkalinizing agents, such as alkali metal bicarbonates.

However, a glutaraldehyde solution, as encompassed by U.S. Patent 3,016,328, demonstrates a pH drift when the container in which it is stored is open and closed or left open during use.

It has now been found that the pH drift of an aqueous solution containing a saturated dialdehyde may be controlled by adding to the dialdehyde solution a particular amount of an alkali metal bicarbonate and/or carbonate and one or more additional buffers, such that the initial aqueous solution has a pH of greater than 7 to about 9.0. The compositions of this invention maintain pH stability for a longer time, and thus the biological activity of the glutaraldehyde solution is prolonged.

It has further been found that the pH stability of a solution containing a saturated dialdehyde may be controlled by reducing the alkali metal bicarbonate and/or carbonate level therein to less than 0.018 molar and adding an additional buffer thereto.

The invention

This invention is directed to an aqueous composition comprising a saturated dialdehyde containing 2 to 6 carbon atoms, an alkali metal bicarbonate and/or carbonate and one or more additional buffers such that the initial aqueous solution has a pH of greater than 7.0 to about 9.0, and wherein the alkali metal carbonates and/or bicarbonates are used in amounts such that the composition contains from 0.006 to 0.018 molar in solution.

The compositions as described herein are suitable for use as disinfectants and/or sporicides for laboratory, surgical, dental, and other equipment.

The saturated dialdehydes which may be used in this invention contain from 2 to 6 carbon atoms and include at least one of the following: malonaldehyde, succinaldehyde, glyoxal, adipaldehyde, and preferably, glutaraldehyde.

The alkali metal carbonates and/or bicarbonates used in this invention may be formed in solution by adding either bicarbonate or carbonate species alone or in combination to form primarily the bicarbonate at the desired pH of the solution.

The alkali metal bicarbonate which may be used herein include one or more alkali metal bicarbonates, such as sodium and potassium bicarbonate. The alkali metal carbonate which is suitable for use herein includes one or more alkali metal carbonates, such as sodium carbonate or potassium carbonate. The bicarbonate and/or carbonate is used in such amounts so as to yield an initial aqueous solution which is from 0.006 to 0.018 molar.

Additional buffer salts may be added so that the initial combination of buffers in the aqueous solution have a pH of greater than 7.0 to about 9.0, preferably from about 7.8 to about 8.5. These additional buffers include phosphates, such as alkali metal phosphates, i.e., sodium phosphate sulfoxylates; secondary or tertiary amines, i.e., diethylaminoethanol, triethylamine; acetates, such as alkali metal acetates, i.e., sodium acetate.

The compositions of this invention may include other ingredients, such as colorants, surfactants and chelating agents.

In a preferred embodiment of this invention, from 0.01 to 5.0 weight percent of a nonionic, cationic or anionic surfactant is added to the composition. Preferred surfactants include nonionic, anionic, and

2

cationic, or amphoteric agents, such as ethoxylates of isomeric linear alcohols or alkyl aryl sulfonates, nonyl phenol ethoxylates, etc. These are well known and described in, for example, U.S. Patents 3,912,450; 3,282,775; and 4,093,744.

Examples

The following examples serve to give specific illustrations of the practice of this invention but they are not intended in any way to limit the scope of this invention.

Control A

The following ingredients were mixed into one liter of water containing glutaraldehyde to yield a 2 percent aqueous glutaraldehyde solution:

2.68 grams of sodium bicarbonate,
(Initial concentration: 0.032 molar)
4.80 grams of sodium hydrogen phosphate, and
(Initial concentration: 0.032 molar)
0.03 grams of sodium dihydrogen phosphate monohydrate
(Initial concentration: 0.002 molar)

The pH of the initial mixture was measured as well as the percent of glutaraldehyde in the mixture. Portions of the mixture were stored in an open container and portions stored in a closed container at room temperature (about 25°C). The pH and percent of glutaraldehyde in the solution were measured after 1, 2 and 5 weeks.

The results in an open container are shown in Table I and in a closed container in Table II.

Example 1

The following ingredients were mixed into one liter of water containing glutaraldehyde to yield a 2 percent aqueous glutaraldehyde solution:

0.893 grams of sodium bicarbonate,
(Initial concentration: 0.011 molar)
4.80 grams of sodium hydrogen phosphate, and
(Initial concentration: 0.032 molar)
0.03 grams of sodium dihydrogen phosphate monohydrate
(Initial concentration: 0.002 molar)

The pH and percent of glutaraldehyde in the mixture were measured as described in Control A. The results are shown in Tables I and II.

Control B

The following ingredients were mixed into one liter of water containing glutaraldehyde to yield a 2 percent aqueous glutaraldehyde solution:

2.68 grams of sodium bicarbonate,
(Initial concentration: 0.032 molar)
4.76 grams of sodium acetate trihydrate, and
(Initial concentration: 0.035 molar)
0.04 grams of sodium hydroxide,
(Initial concentration 0.001 molar)

The sodium hydroxide was added to the solution to adjust its pH to 8.0.
The pH and percent of glutaraldehyde in the mixture were measured as described in Control A. The results are shown in Tables I and II.

Example 2

The following ingredients were mixed into one liter of water containing glutaraldehyde to yield a 2 percent aqueous glutaraldehyde solution:

0.893 grams of sodium bicarbonate,
(Initial concentration: 0.011 molar)
4.76 grams of sodium acetate trihydrate, and
(Initial concentration: 0.035 molar)
0.04 grams of sodium hydroxide,
(Initial concentration: 0.001 molar)
The sodium hydroxide was added to its solution to adjust its pH to 8.0.

The pH and percent of glutaraldehyde in the mixture were measured as described in Control A. The results are shown in Tables I and II.

The data show that in closed containers (Table II) with the concentrations of bicarbonate shown, the pH of the resulting aged aqueous solutions are relatively stable. However, in open containers (Table I), with high levels of bicarbonate, there is a dramatic increase of pH with time which results in a substantial decline in the concentration of glutaraldehyde.

TABLE I

| Example | Buffers in the composition (grams) | | | | Data in open container | | | | | | | |
| | Sodium bicarbonate | Sodium hydrogen phosphate | Sodium dihydrogen phosphate | Sodium acetate trihydrate | Initial | | One week | | Two weeks | | Five weeks | |
| | | | | | pH | Glutaraldehyde (%) | pH | Glutaraldehyde (%) | pH | Glutaraldehyde (%) | pH | Glutaraldehyde (%) |
|---|---|---|---|---|---|---|---|---|---|---|---|---|
| Control A | 2.68 | 4.80 | 0.03 | — | 7.9 | 2.4 | — | — | 8.5 | 1.0 | 8.4 | 0.5 |
| 1 | 0.893 | 4.80 | 0.03 | — | 7.8 | 2.4 | — | — | 8.1 | 1.7 | 7.8 | 1.2 |
| Control B | 2.68 | — | — | 4.76 | 8.0* | 2.4 | 8.8** | 1.0 | 8.8 | 0.8 | — | — |
| 2 | 0.893 | — | — | 4.76 | 8.0* | 2.4 | 8.0** | 1.9 | 8.0 | 1.8 | — | — |

*Initial pH adjusted to 8.0 with sodium hydroxide.     **These samples were measured after 9 days.

TABLE II

| Example | Buffers in the composition (grams) | | | | Data in closed container | | | | | | | |
| | Sodium bicarbonate | Sodium hydrogen phosphate | Sodium dihydrogen phosphate | Sodium acetate trihydrate | Initial | | One week | | Two weeks | | Five weeks | |
| | | | | | pH | Glutaraldehyde (%) | pH | Glutaraldehyde (%) | pH | Glutaraldehyde (%) | pH | Glutaraldehyde (%) |
|---|---|---|---|---|---|---|---|---|---|---|---|---|
| Control A | 2.68 | 4.80 | 0.03 | — | 7.9 | 2.4 | 7.7 | — | 7.7 | 1.9 | 7.6 | 1.6 |
| 1 | 0.893 | 4.80 | 0.03 | — | 7.8 | 2.4 | 7.8 | — | 7.7 | 1.9 | 7.5 | 1.3 |
| Control B | 2.68 | — | — | 4.76 | 8.0* | 2.4 | 8.0** | 1.9 | 7.9 | 1.8 | 7.6 | 1.7 |
| 2 | 0.893 | — | — | 4.76 | 8.0* | 2.4 | 7.5** | 2.2 | 7.6 | 2.1 | 7.3 | 2.0 |

* Initial pH adjusted to 8.0 with sodium hydroxide.     ** These samples were measured after 9 days.

In the following Examples and Control varying amounts of sodium bicarbonate were added to a 2 percent aqueous glutaraldehyde solution. To the solution of the Examples and Controls were added constant additional amounts of sodium buffer salts to control the initial pH.

Control C

The following ingredient was mixed into one liter of water containing glutaraldehyde to yield a 2 percent aqueous glutaraldehyde solution:

   0.30 weight percent of sodium bicarbonate
        (Initial concentration: 0.036 molar)

The pH of the mixture was measured after preparation. Portions of the mixture was stored in an open container and portions in a closed container at room temperature (about 25°C) for four weeks. The pH was measured at the end of four weeks.

The results are shown in Table III.

Example 3

The following ingredient was mixed into one liter of water containing glutaraldehyde to yield a 2 percent aqueous glutaraldehyde solution:

   0.15 weight percent of sodium bicarbonate
        (Initial concentration: 0.018 molar)

The mixture was tested as described in Control C.

The results are shown in Table III.

Example 4

The following ingredient was mixed into one liter of water containing glutaraldehyde to yield a 2 percent aqueous glutaraldehyde solution:

   0.08 weight percent of sodium bicarbonate
        (Initial concentration: 0.011 molar)

The mixture was tested as described in Control C.

The results are shown in Table III.

Control D

This was a 2 percent aqueous glutaraldehyde solution containing no sodium bicarbonate.

The glutaraldehyde solution was tested as described in Control C.

The results are shown in Table III.

The data in Table III shows that the pH change between the open and closed container is dramatically reduced when using the preferred bicarbonate range as shown in Examples 3 and 4. Higher levels of bicarbonate, as shown in Control C, result in a larger pH drift whereas without bicarbonate, Control D, the pH drifts to an unacceptable acidic pH in open containers.

TABLE III

| Example | Sodium bicarbonate (wt.%) | Initial pH* | pH after 4 weeks in open container | pH after 4 weeks in closed container | (Open-Closed) |
|---------|---------|---------|---------|---------|---------|
| Control C | 0.30 | 7.9 | 8.6 | 7.3 | 1.3 |
| 3 | 0.15 | 7.9 | 8.2 | 7.2 | 1.0 |
| 4 | 0.08 | 7.9 | 7.5 | 7.0 | 0.5 |
| Control D | — | 7.9 | 5.3** | 6.8 | — |

\* Constant amounts of sodium buffer salts were added to control the initial pH.
\*\* Unusable since too acidic.

In the following Table IV, which simulates use conditions, the effect of opening and closing a container on the pH of a 2 percent glutaraldehyde solution containing different amounts of sodium bicarbonate is demonstrated. The formulation of Example 5 contained 2.68 grams of sodium bicarbonate (Initial concentration: 0.032 molar) while the formulation of Control E contained 0.893 grams of sodium bicarbonate (Initial concentration 0.011 molar). The formulations of Example 5 and Control E contained constant additional amounts of sodium buffer salts to control the initial pH.

The container was stored at room temperature (about 25°C) for 14 days. The days the container remained opened and closed is shown in Table IV. The pH of the solution was measured at that point in time. ΔpH in Table IV is the difference between the highest and lowest pH values. The data shows that the drift in pH, i.e., ΔpH, is lower with the bicarbonate levels of this invention.

TABLE IV

| Container | Closed | Opened | Closed | Opened | Closed | Opened | ΔpH |
|---|---|---|---|---|---|---|---|
| Time (days) | 0 | 3 | 2 | 2 | 3 | 2 | 2 | |
| Elapsed time (days) | 0 | 3 | 5 | 7 | 10 | 12 | 14 | |
| Example | | | | pH | | | | |
| 5 | 8.05 | 7.86 | 8.05 | 7.86 | 8.05 | 7.86 | 7.98 | 0.19 |
| Control E | 8.05 | 7.80 | 8.58 | 8.14 | 8.60 | 8.05 | 8.44 | 0.80 |

**Claims**

1. An aqueous composition comprising a saturated dialdehyde containing 2 to 6 carbon atoms, an alkali metal bicarbonate and/or carbonate and one or more additional buffers such that the initial aqueous solution has a pH of greater than 7.0 to about 9.0, characterized in that the alkali metal carbonate and/or bicarbonate is provided in amounts such that the concentration thereof is from 0.006 to 0.018 molar.

2. A composition as defined in claim 1 wherein the dialdehyde is glutaraldehyde.

3. A composition as defined in claims 1 or 2 wherein the additional buffer is selected from one or more of the following: a phosphate, a sulfoxylate, a secondary or tertiary amine, or an acetate.

4. The composition as defined in any of the preceding claims wherein the alkali metal bicarbonate and/or carbonate is selected from sodium bicarbonate, potassium bicarbonate, sodium carbonate and potassium carbonate or mixtures thereof.

5. A composition as defined in any of the preceding claims wherein the pH is from about 7.8 to about 8.5.

6. A composition as defined in any of the preceding claims which contains a surfactant.

7. A composition as defined in any of the preceding claims wherein the alkali metal carbonate and/or bicarbonate is provided in amounts such that the concentration thereof is from about 0.006 to about 0.011 molar.

8. A method for disinfecting and/or sterilizing medical, dental, surgical or other objects by contacting said object with an aqueous sporicidal composition comprising a saturated dialdehyde containing from 2 to 6 carbon atoms, an alkali metal bicarbonate and/or carbonate and one or more additional buffers such that the initial aqueous solution has a pH of greater than 7.0 to about 9.0, characterized in that the alkali metal carbonate and/or bicarbonate is provided in amounts such that the concentration thereof is from 0.006 to 0.018 molar.

**Patentansprüche**

1. Wässrige Mischung, enthaltend einen gesättigten Dialdehyd mit 2 bis 6 Kohlenstoffatomen, ein Alkalimetallbicarbonat und/oder -carbonat und einen oder mehrere zusätzliche Puffer, so daß die anfängliche wässrige Lösung einen pH größer als 7,0 bis etwa 9,0 hat, dadurch gekennzeichnet, daß das Alkalimetallcarbonat und/oder bicarbonat in solchen Mengen vorgesehen ist, daß dessen Konzentration 0,006 bis 0,018 molar ist.

2. Mischung nach Anspruch 1, in welcher der Dialdehyd Glutaraldehyd ist.

3. Mischung nach Anspruch 1 oder 2, in welcher der zusätzliche Puffer aus einem oder mehreren der folgenden Materialien ausgewählt ist: ein Phosphat, ein Sulfoxylat, ein sek- oder tert-Amin oder ein Acetat.

4. Mischung nach einem der vorhergehenden Ansprüche, in welcher das Alkalibicarbonat und/oder carbonat ausgewählt ist aus Natriumbicarbonat, Kaliumbicarbonat, Natriumcarbonat und Kaliumcarbonat oder Mischungen derselben.

5. Mischung nach einem der vorhergehenden Ansprüche, in welcher der pH von etwa 7,8 bis etwa 8,5 beträgt.

6. Mischung nach einem der vorhergehenden Ansprüche, die ein oberflächenaktives Mittel enthält.

7

7. Mischung nach einem der vorhergehenden Ansprüche, in welcher das Alkalicarbonat und/oder -bicarbonat in solchen Mengen vorgesehen ist, daß dessen Konzentration von etwa 0,006 bis etwa 0,011 molar ist.

8. Verfahren zum Desinfizieren und/oder Sterilisieren medizinischer, Dental-, chirurgischer oder anderer Gegenstände durch In-Berührung-bringen des Gegenstandes mit einer wässrigen sporiziden Mischung, die einen gesättigten Dialdehyd mit 2 bis 6 Kohlenstoffatomen, ein Alkalimetallbicarbonat und/oder -carbonat und einen oder mehrere zusätzliche Puffer enthält, so daß die anfängliche wässrige Lösung einen pH größer als 7,0 bis etwa 9,0 hat, dadurch gekennzeichnet, daß das Alkalimetallcarbonat und/oder -biscarbonat in solchen Mengen vorgesehen ist, daß dessen Konzentration von 0,006 bis 0,018 molar ist.

## Revendications

1. Composition aqueuse comprenant un dialdéhyde saturé contenant 2 à 6 atomes de carbone, un bicarbonate et/ou un carbonate de métal alcalin et un ou plusieurs tampons additionnels afin que la solution aqueuse initiale ait un pH allant d'une valeur supérieure à 7,0 à environ 9,0, caractérisée en ce que le carbonate et/ou le bicarbonate de métal alcalin sont présents en quantités choisies de manière que leur concentration molaire ait une valeur de 0,006 à 0,018.

2. Composition suivant la revendication 1, dans laquelle le dialdéhyde est le glutaraldéhyde.

3. Composition suivant les revendications 1 ou 2, dans laquelle le tampon additionnel consiste en une ou plusieurs des substances suivantes: un phosphate, un sulfoxylate, une amine secondaire ou tertiaire ou un acétate.

4. Composition suivant l'une quelconque des revendications précédentes, dans laquelle le bicarbonate et/ou le carbonate de métal alcalin sont choisis entre le bicarbonate de sodium, le bicarbonate de potassium, le carbonate de sodium et le carbonate de potassium ou leurs mélanges.

5. Composition telle que définie dans l'une quelconque des revendications précédentes, dans laquelle le pH va d'environ 7,8 à environ 8,5.

6. Composition telle que définie dans l'une quelconque des revendications précédentes, qui contient un surfactant.

7. Composition telle que définie dans l'une quelconque des revendications précédentes, dans laquelle le carbonate et/ou le bicarbonate de métal alcalin sont présents en quantités choisies de manière que leur concentration molaire ait une valeur d'environ 0,006 à environ 0,011.

8. Procédé pour désinfecter et/ou stériliser des objets médicaux, dentaires, chirurgicaux ou autres objets par mise en contact desdits objets avec une composition aqueuse sporicide comprenant un dialdéhyde saturé contenant 2 à 6 atomes de carbone, un bicarbonate et/ou un carbonate de métal alcalin et un ou plusieurs autres tampons, de manière que la solution aqueuse initiale ait un pH allant d'une valeur supérieure à 7,0 à une valeur d'environ 9,0, caractérisé en ce que le carbonate et/ou le bicarbonate de métal alcalin sont présents en quantités telles que leur concentration molaire ait une valeur de 0,006 à 0,018.